# EUROPEAN PATENT APPLICATION

(11) **EP 2 535 002 A1**
(43) Date of publication of application: **19.12.2012**
(21) Application number: 12171636.9
(22) Date of filing: 12.06.2012
(51) Int. Cl.: A61B 6/00, A61B 6/02, G06T 7/00, G06T 11/00

(54) **Radiological image radiographing apparatus and method**

(30) Priority: 17.06.2011 JP 2011134732
(71) Applicant: Fujifilm Corporation, Minato-ku Tokyo 106-8620 (JP)
(72) Inventor: Nakayama, Hiroki, Kanagawa, 258-8538 (JP)
(74) Representative: Klunker . Schmitt-Nilson . Hirsch

(57) **Abstract**

Deterioration of the image quality of a displayed image is prevented when displaying one image, such as a tomographic image or a stereoscopic image, based on a plurality of radiological images acquired by performing radiographing multiple times in order to display a tomographic image or a stereoscopic image. A feature amount calculation unit calculates feature amounts representing the image quality, such as density histograms, for a plurality of radiological images for generating a tomographic image acquired by tomosynthesis. A determination unit compares the feature amounts of the plurality of radiological images and based on the comparison result, and then determines whether or not an abnormal radiological image with different image quality is present in the plurality of radiological images. When an abnormal radiological image is present, a reconstruction unit generates a tomographic image by performing reconstruction after excluding the abnormal radiological image from the plurality of radiological images.

## Description

### BACKGROUND OF THE INVENTION

1. **Field of the Invention**

The present invention relates to a radiological image radiographing apparatus and method for acquiring a plurality of radiological images by radiographing a subject from a plurality of different radiographing directions.

2. **Description of the Related Art**

An apparatus which displays a stereoscopic image (a three-dimensional image or a stereo image) based on two radiological images including information regarding parallax between both the left and right eyes has been proposed as an apparatus which displays a medical image, such as a radiological image. Such an apparatus irradiates a subject from different directions, detects radiation transmitted through the subject using a radiation detector to acquire a plurality of radiological images with parallax with respect to one another, and displays a stereoscopic image based on these radiological images that is able to be viewed stereoscopically. By displaying a stereoscopic image as described above, diagnosis can be performed more easily since a radiological image can be observed with a sense of depth.

In addition, in radiological image radiographing apparatuses, in order to observe an affected part in more detail, tomosynthesis in which a radiation source is moved to irradiate a subject from a plurality of different directions and the images acquired are added to obtain an image which emphasizes a desired cross section, has been proposed. In tomosynthesis, a plurality of radiological images are acquired by radiographing a subject at a plurality of irradiation positions with different irradiation angles while moving a radiation source in parallel to a radiation detector or moving the radiation source so as to draw the arc of a circle or ellipse according to the characteristics or a required tomographic image of a radiographing apparatus. These radiological images are reconstructed using a back projection method, such as a simple back projection method or a filter back projection method, to generate a tomographic image.

Here, when acquiring a plurality of radiological images of the structure of the human body, a method of performing image processing on each radiological image in order to make the image qualities of the plurality of radiological images equal has been proposed (refer to JP2001-092948A). In addition, in mammography, a method of acquiring index values for comparison of radiological images of the left and right breasts, determining whether or not a difference between the index values is within a predetermined range, and notifying the result has been proposed (refer to JP2010-155017A). In addition, when generating a tomographic image from a plurality of radiological images with respect to the circuit pattern, a method has been proposed of detecting the position of the circuit pattern by comparing the plurality of radiological images using histograms, spatial frequencies, edge information, and the like with respect to the plurality of radiological images (refer to JP2008-026334A).

### SUMMARY OF THE INVENTION

When performing tomosynthesis or performing radiographing multiple times in order to generate a stereoscopic image, the amount of radiation at the time of radiographing may be reduced, for example, due to deterioration of the condition of the radiation source during radiographing or the like. In such a case, a radiological image acquired with a low amount of radiation has a low density compared with other radiological images. Accordingly, if a tomographic image or a stereoscopic image is generated using such a radiological image, the image quality of a generated image deteriorates.

Since the above method disclosed in JP2001-092948A is a method of performing image processing on a radiological image, deterioration of the image quality of the generated image can be prevented. However, when the method disclosed in JP2001-092948A is applied to the generation of a tomographic image or a stereoscopic image based on a plurality of radiological images, processing efficiency is down. In addition, the method disclosed in JP2010-155017A only compares images using the index values of radiological images. In addition, the method disclosed in JP2008-026334A only compares images using information from histograms and the like in order to detect the position of a circuit pattern.

The present invention has been made in view of the above-mentioned problems and an object of the present invention is to prevent deterioration of the image quality of a displayed image when displaying one image such as a tomographic image or a stereoscopic image based on a plurality of radiological images acquired by performing radiographing multiple times in order to display a tomographic image or a stereoscopic image.

According to an aspect of the present invention, there is provided a radiological image radiographing apparatus including: an irradiation unit that radiates radiation to a subject; a radiation detection unit that detects radiation, which is radiated from the irradiation unit to be then transmitted through the subject, to acquire a radiological image of the subject; a radiographing control unit that controls the irradiation unit and the radiation detection unit to radiate the radiation to the subject from a plurality of radiographing directions and to acquire a plurality of radiological images with different directions of radiographing by irradiation of the radiation; a feature amount calculation unit that calculates feature amounts representing an image quality of each of the plurality of radiological images; and a determination unit that compares the feature amounts of the plurality of radiological images and based on the comparison result, and then determines whether or not an abnormal radiological image with different image quality is present in the plurality of radiological images.

In addition, in the radiological image radiographing apparatus according to the aspect of the present invention, the feature amount calculation unit may be constituted for calculating feature amounts based on at least one of density histograms, spatial frequencies, edge information, S/N ratios, and contrast of the plurality of radiological images.

More specifically, for density histograms, the signal value at the peak of the density histogram, the size of the distribution range of the density histogram, the correlation value of the density histogram between a plurality of radiological images, information indicating whether or not there is a different peak from those in density histograms of many other radiological images, and the like may be used as feature amounts. In addition, "many radiological images" means a number of radiological images which exceeds the majority of a plurality of radiological images. In addition, for the spatial frequencies, a spectrum with a specific spatial frequency component or the integrated value of the spectrum may be used as a feature amount. In addition, for the edge information, the edge may be extracted from a radiological image and the size of the pixel values of the extracted edge may be used. The S/N ratio can be calculated using the pixel values of a portion of a marker image in a radiological image acquired by radiographing a marker, such as metal, together with a subject. The contrast can be calculated using a difference between the pixel values of the portion of the marker image and the pixel values of an element dropping out portion of the radiological image, to which radiation is directly radiated to the radiation detector.

In addition, in the radiological image radiographing apparatus according to the aspect of the present invention, the determination unit may be constituted for determining whether or not an abnormal radiological image is present in the plurality of radiological images by identifying a radiological image with feature amounts, which are different from those of other radiological images by a predetermined threshold value or more, as an abnormal radiological image.

In addition, in the radiological image radiographing apparatus according to the aspect of the present invention, a reconstruction unit that generates a tomographic image on a desired sectional surface of the subject by reconstructing the plurality of radiological images when the plurality of radiological images are acquired in order to generate a tomographic image, may be further provided.

In this case, when an abnormal radiological image is present, the reconstruction unit may be constituted for generating a tomographic image by reconstructing other radiological images excluding the abnormal radiological image in the plurality of radiological images.

Moreover, in this case, the reconstruction unit may be constituted for stopping reconstruction when the abnormal radiological image is present.

In addition, in the radiological image radiographing apparatus according to the aspect of the present invention, display unit that, when the plurality of radiological images are two radiological images acquired in order to display a stereoscopic image, displays the stereoscopic image based on the two radiological images, may be further provided.

In this case, the display unit may be constituted for stopping display of the stereoscopic image when the abnormal radiological image is present.

In addition, in the radiological image radiographing apparatus according to the aspect of the present invention, warning unit for giving a warning when an abnormal radiological image is present may be further provided.

According to another aspect of the present invention, there is provided a radiological image radiographing method in a radiological image radiographing apparatus including an irradiation unit that radiates radiation to a subject, a radiation detection unit that detects radiation, which is radiated from the irradiation unit to be then transmitted through the subject, to acquire a radiological image of the subject, and a radiographing control unit that controls the irradiation unit and the radiation detection unit to radiate the radiation to the subject from a plurality of radiographing directions and to acquire a plurality of radiological images with different directions of radiographing by irradiation of the radiation. The radiological image radiographing method includes: calculating feature amounts indicating respective image qualities of the plurality of radiological images; comparing the feature amounts of the plurality of radiological images; and determining, based on the comparison result, whether or not an abnormal radiological image with different image quality is present in the plurality of radiological images.

According to the aspect of the present invention, a plurality of radiological images are acquired, feature amounts indicating respective image qualities of the plurality of radiological images are calculated, the feature amounts of the plurality of radiological images are compared, and based on the comparison result, it is determined whether or not an abnormal radiological image with different image quality is present in the plurality of radiological images. For this reason, in a case where there is an abnormal radiological image, when a tomographic image is generated using a plurality of radiological images or a stereoscopic image is displayed, it is possible to do so without using the abnormal radiological image or to stop the display of a tomographic image or a stereoscopic image. As a result, deterioration of the image quality of an image displayed based on the plurality of radiological images can be prevented.

In addition, since the feature amounts are calculated based on any of the density histograms, spatial frequencies, edge information, S/N ratios, and contrast of the plurality of radiological images, the feature amounts can be calculated with a simple operation.

In addition, since a radiological image with feature amounts, which are different from those of many other radiological images by a predetermined threshold value or more, is identified as an abnormal radiological image, the presence of an abnormal radiological in the plurality of radiological images can be easily determined.

In addition, the presence of an abnormal radiological image can be notified by giving a warning when there is an abnormal radiological image.

### BRIEF DESCRIPTION OF THE DRAWINGS

Fig. 1 is a view showing the schematic configuration of a radiological image radiographing apparatus according to a first embodiment of the present invention.

Fig. 2 is a view when an arm unit of the radiological image radiographing apparatus shown in Fig. 1 is seen from the right side of Fig. 1.

Fig. 3 is a block diagram showing the schematic internal configuration of a computer of the radiological image radiographing apparatus shown in Fig. 1.

Fig. 4 is a view showing a plurality of radiographing directions.

Fig. 5 is a view showing a reconstruction range of a tomographic image.

Fig. 6 is a view for explaining a change in the signal value at the peak position of a density histogram.

Fig. 7 is a flow chart showing the processing performed in the first embodiment.

Fig. 8 is a flow chart showing the processing performed in a second embodiment.

Fig. 9 is a flow chart showing the processing performed in a third embodiment.

Fig. 10 is a view showing a radiological image including a marker image.

### DESCRIPTION OF THE PREFERRED EMBODIMENTS

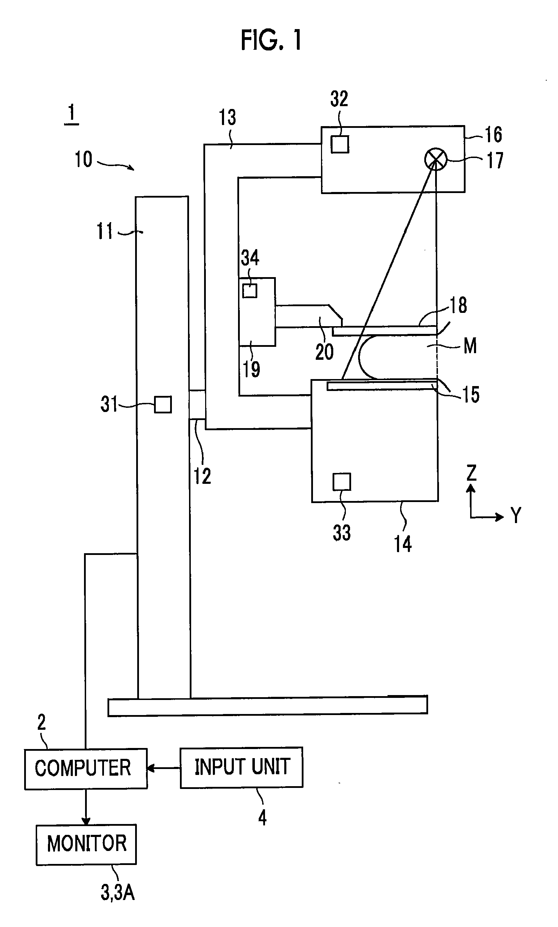

Hereinafter, embodiments of the present invention will be described with reference to the accompanying drawings. Fig. 1 is a view showing the schematic configuration of a radiological image radiographing apparatus according to a first embodiment of the present invention. A radiological image radiographing apparatus 1 acquires a plurality of radiological images by radiographing a breast M from different radiographing directions in order to generate a tomographic image by performing tomosynthesis of the breast M and also to generate a stereo image for stereoscopic viewing of a radiological image of the breast M. As shown in Fig. 1, the radiological image radiographing apparatus 1 includes a radiographing unit 10, a computer 2 connected to the radiographing unit 10, and a monitor 3 and an input unit 4 connected to the computer 2.

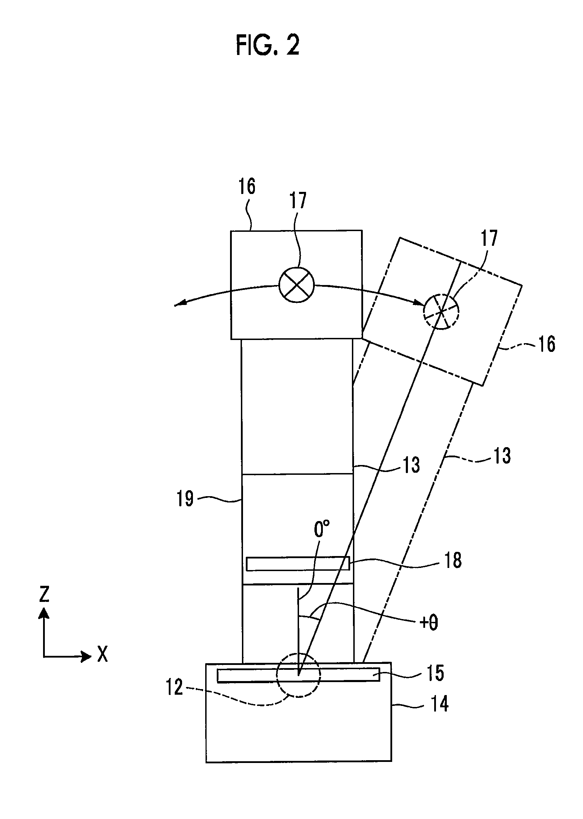

The radiographing unit 10 includes a pedestal 11, a rotary shaft 12 which can be rotated and moved up and down (in a Z direction) with respect to the pedestal 11, and an arm unit 13 connected to the pedestal 11 through the rotary shaft 12. In addition, Fig. 2 shows the arm unit 13 when viewed from the right side in Fig. 1.

The arm unit 13 is C-shaped. A radiography platform 14 is fixed to one end of the arm unit 13, and an irradiation unit 16 is fixed to the other end so as to face the radiography platform 14. Rotation and up-and-down movement of the arm unit 13 are controlled by an arm controller 31 provided in the pedestal 11.

A radiation detector 15, such as a flat panel detector, and a detector controller 33 which controls reading of a charge signal from the radiation detector 15, are provided inside the radiography platform 14.

In addition, a circuit board on which a charge amplifier that converts a charge signal read from the radiation detector 15 into a voltage signal, a correlated double sampling circuit that samples a voltage signal output from the charge amplifier, an AD converter that converts a voltage signal into a digital signal, and the like are provided is placed inside the radiography platform 14. In addition, a radiation detection unit is formed by the radiation detector 15, the detector controller 33, and devices related to radiation detection including the circuit board and the like.

In addition, the radiography platform 14 is configured to be able to rotate with respect to the arm unit 13. Accordingly, even when the arm unit 13 rotates with respect to the pedestal 11, the direction of the radiography platform 14 can be fixed with respect to the pedestal 11.

The radiation detector 15 can perform recording and reading of a radiological image repeatedly. A so-called direct-type radiation detector which generates an electric charge by direct reception of radiation may be used, or a so-called indirect-type radiation detector which converts radiation into visible light and then converts the visible light into a charge signal may be used. In addition, as a method of reading a radiological image signal, a so-called TFT reading method in which a radiological image signal is read by switching on or off a TFT (thin film transistor) or a so-called optical reading method in which a radiological image signal is read by irradiation of reading light, is preferably used. However, other methods may be used without being limited to these methods.

A radiation source 17 and a radiation source controller 32 are contained in the irradiation unit 16. The radiation source controller 32 controls the irradiation timing of radiation from the radiation source 17 and the radiation generating conditions (tube current, time, tube voltage, and the like) in the radiation source 17.

In addition, a compression plate 18 provided above the radiography platform 14 to compress a breast, a support unit 20 which supports the compression plate 18, and a moving mechanism 19 which moves the support unit 20 up and down (in the Z direction) are provided in the middle of the arm unit 13. The position and the pressure of the compression plate 18 are controlled by a compression plate controller 34.

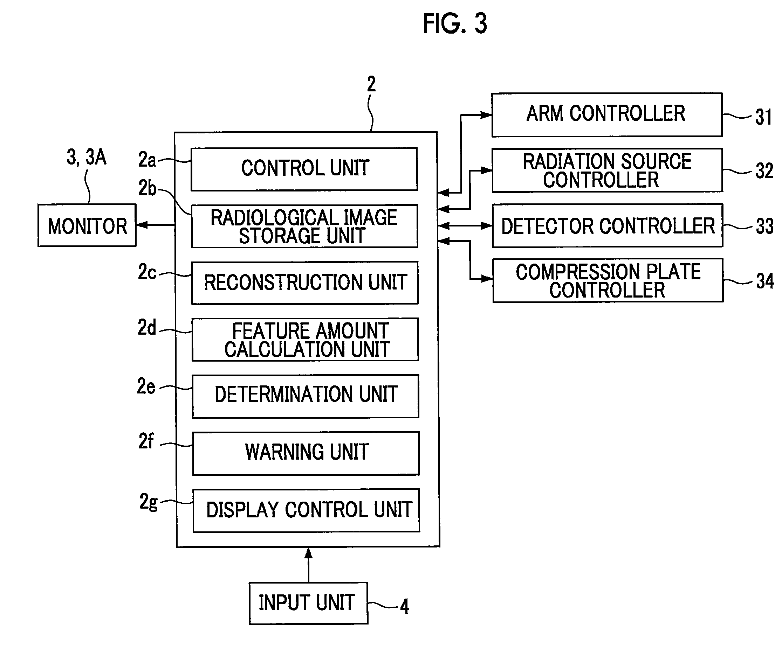

The computer 2 includes a central processing unit (CPU) and a storage device, such as a semiconductor memory, a hard disk, or an SSD. By these hardware components, a control unit 2a, a radiological image storage unit 2b, a reconstruction unit 2c, a feature amount calculation unit 2d, a determination unit 2e, a warning unit 2f, and a display control unit 2g shown in Fig. 3 are constituted.

The control unit 2a outputs predetermined control signals to various kinds of controllers 31 to 34 to control the entire apparatus, and includes a radiographing control section which controls the irradiation unit and the radiation detection unit so as to acquire a plurality of radiological images with different radiographing directions by radiation.

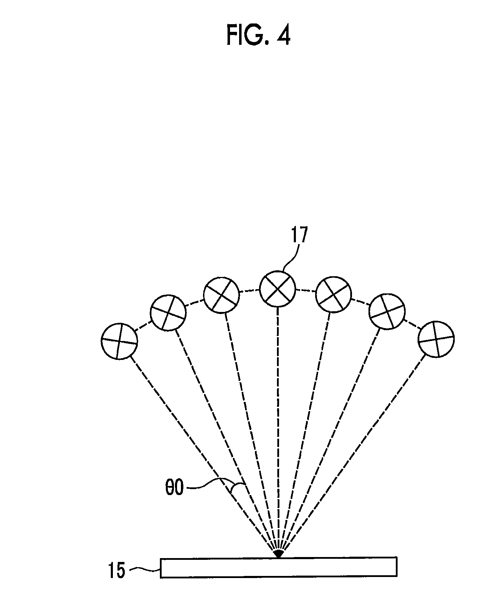

The radiological image storage unit 2b stores a plurality of radiological images that the radiation detector 15 detects by performing radiographing from a plurality of radiographing directions that have gaps of predetermined angles θ0 as shown in Fig. 4 while rotating the arm unit 13. In addition, in the first embodiment, a plurality of tomographic images of a plurality of cross sections of the breast M, which are generated from the plurality of radiological images, in its depth direction (Z direction) are generated, and the edge is extracted from the plurality of tomographic images to generate a plurality of tomographic edge images. In addition, the radiological image radiographing apparatus 1 according to the first embodiment can display a stereo image using a radiological image, which is acquired by radiographing the breast from the direction of 0° (that is, a direction perpendicular to the radiation detector 15), and a radiological image with a different radiographing direction from the radiological image with the direction of 0°, among a plurality of radiological images. In addition, regarding the rotation direction of the arm unit 13, rotation to the right in Fig. 2 is a forward direction.

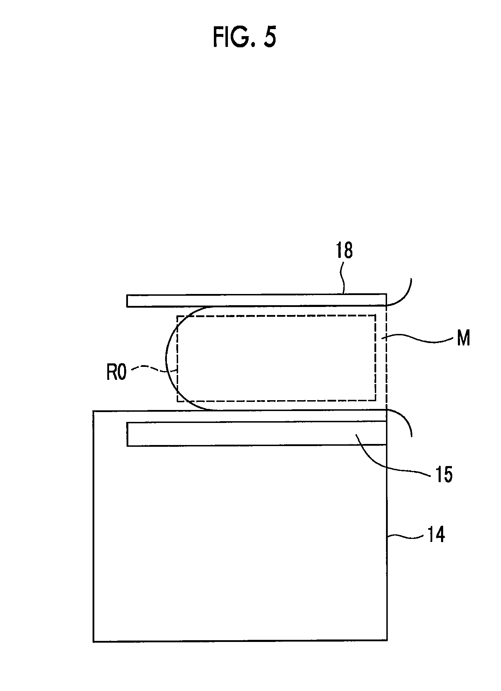

The reconstruction unit 2c generates a plurality of tomographic images corresponding to a plurality of cross sections of the breast M in its depth direction by reconstructing a plurality of radiological images stored in the radiological image storage unit 2b. Specifically, the reconstruction unit 2c generates a plurality of tomographic images by reconstructing these radiological images using a back projection method, such as a simple back projection method or a filter back projection method. In addition, the reconstruction unit 2c generates a plurality of tomographic images of a plurality of cross sections, which is parallel to the detection plane of the radiation detector 15, within a reconstruction range R0 set in advance in the breast M as shown in Fig. 5.

In addition, the reconstruction unit 2c changes a method of reconstruction according to the determination result of the determination unit 2e which will be described later.

The feature amount calculation unit 2d calculates feature amounts representing the image quality of each of the plurality of radiological images stored in the radiological image storage unit 2b. In the present embodiment, the feature amount calculation unit 2d calculates the feature amounts representing the image quality based on at least one of density histograms, spatial frequencies, and edge information of a plurality of radiological images.

Density histograms is calculated by plotting the signal value on the horizontal axis and the frequency of the signal value on the vertical axis in each of the plurality of radiological images. In addition, any of the signal value at the peak position of the density histogram, the size of the distribution range of a density histogram, the correlation value of the density histogram between a plurality of radiological images, and information indicating whether or not there is a different peak from those in density histograms of many other radiological images, is calculated as feature amounts. In addition, it is assumed that all possible correlation values between a plurality of radiological images are calculated as correlation values of the density histogram of the plurality of radiological images.

For the spatial frequency, a Fourier transform of a radiological image is performed, and a spectrum with a predetermined spatial frequency is calculated as the feature amount. Alternatively, the spectrum is integrated, and the integral value is calculated as the feature amount.

For the edge information, edge pixels are extracted from a radiological image using an edge extraction filter, such as a Sobel filter or a Laplacian filter. In addition, the pixel values of an edge pixel is calculated as the feature amount.

The determination unit 2e compares the feature amounts calculated by the feature amount calculation unit 2d between the plurality of radiological images, and based on the comparison result, and then determines whether or not an abnormal radiological image with different image quality is present in the plurality of radiological images by identifying an abnormal radiological image with different image quality. In addition, when it is determined that there is an abnormal radiological image, the determination unit 2e outputs the determination result to the reconstruction unit 2c and the warning unit 2f. When it is determined that there is no abnormal radiological image, the determination unit 2e outputs the determination result only to the reconstruction unit 2c.

In general, only a small number of abnormal radiological images are included in a plurality of radiological images. However, it is possible for many abnormal radiological images to be included. When the feature amounts are compared in such a case, the number of radiological images with different feature amounts may not so differ from the number of other radiological images. In such a case, it is assumed that the determination unit 2e determines radiological images less than a majority as abnormal radiological images.

Here, when the feature amounts are the signal value at the peak position of the density histogram and the size of the distribution range, differences between all possible feature amounts of the plurality of radiological images are calculated, and a radiological image with feature amounts which are different from those of other radiological images by the predetermined threshold value or more is identified as an abnormal radiological image.

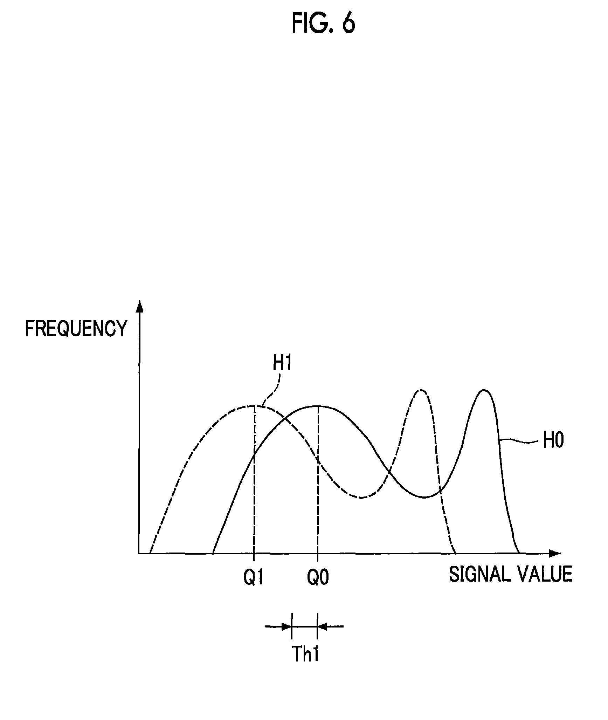

For example, when the feature amount is a signal value at the peak position of the density histogram, assuming that the abnormality occurs in the radiation source 17 at the time of radiographing of a certain radiological image and the amount of radiation radiated to the breast M is reduced, the signal value of the peak position of the density histogram is low. Fig. 6 is a view for explaining a change in the signal value at the peak position of a density histogram. In addition, in Fig. 6, H0 indicates a density histogram of a radiological image acquired by radiographing when there is no abnormality, and H1 indicates a density histogram of a radiological image acquired by radiographing when there is an abnormality. As shown in Fig. 6, when the signal value Q0 at the peak position of the density histogram H0 is compared with the signal value Q1 at the peak position of the density histogram H1, the signal value Q1 is less than the signal value Q0 by an amount exceeding the threshold value Th1. In such a case, since density histograms of many of the plurality of radiological images are H0, a radiological image which has the density histogram H1, in which the signal value at the peak position is low by an amount exceeding the threshold value Th1, is identified as an abnormal radiological image.

In addition, when the feature amount is the size of the distribution range of a density histogram, the maximum value or the minimum value of the distribution range is compared between a plurality of radiological images, for example, and a radiological image with a maximum value or a minimum value, which is different from those of many of the plurality of radiological images by an amount exceeding the predetermined threshold value, is identified as an abnormal radiological image.

On the other hand, when the feature amount is a correlation value of a density histogram, correlation values of many radiological images are compared, and a radiological image whose correlation value is small by an amount exceeding the predetermined threshold value or more is identified as an abnormal radiological image. In addition, when the feature amount is information indicating whether or not there is a different peak from those in density histograms of many other radiological images, a radiological image with a different peak from those in the density histograms of other radiological images is identified as an abnormal radiological image.

In addition, if the amount of radiation when acquiring a radiological image is small when the feature amount is a feature amount based on the spatial frequency, a spectrum of a frequency component corresponding to noise or its integral value is large since the noise is noticeable. For this reason, the determination unit 2e identifies a radiological image with feature amounts, which are different from those of many radiological images by an amount exceeding the predetermined threshold value, as an abnormal radiological image.

In addition, if the amount of radiation when acquiring a radiological image is small when the feature amount is edge information, a radiological image with feature amounts which are different from those of many other radiological images by an amount exceeding the predetermined threshold value is identified as an abnormal radiological image since the density of the edge is low.

In addition, when it is determined that there is an abnormal radiological image, the reconstruction unit 2c excludes the abnormal radiological image from the reconstruction of a tomographic image and performs the reconstruction using only radiological images other than the abnormal radiological image. When it is determined that there is no abnormal radiological image, the reconstruction unit 2c performs the reconstruction using all radiological images.

The warning unit 2f gives a warning when the determination result representing that there is an abnormal radiological image is input from the determination unit 2e. As a warning, the presence of an abnormal radiological image may be displayed on the monitor 3, or a sound indicating that there is an abnormal radiological image may be output from a speaker (not shown). Alternatively, a specific beep may be output, or the display using the monitor 3 and the sound output may be combined.

The display control unit 2g displays a plurality of radiological images and tomographic images on the monitor 3.

The input unit 4 includes a keyboard or a pointing device, such as a mouse, and receives from an operator an input of radiographing conditions, an input of a radiographing start instruction, and the like.

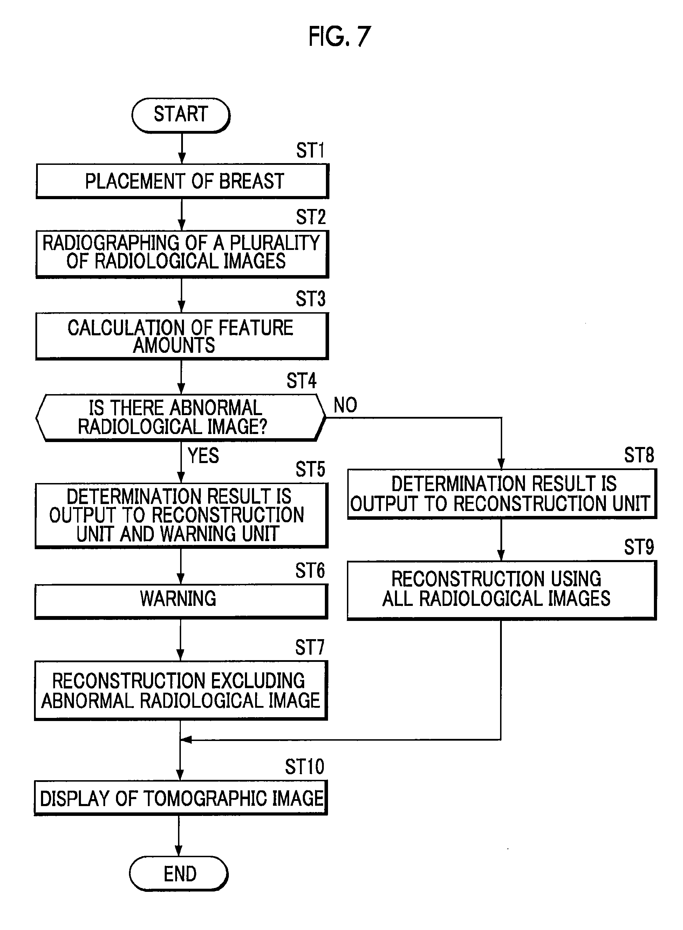

Next, processing performed in the first embodiment will be described. Fig. 7 is a flow chart showing the processing performed in the first embodiment. First, the breast M of a patient is placed on the radiography platform 14 and is compressed with predetermined pressure by the compression plate 18 (step ST1). Then, various radiographing conditions are input through the input unit 4 and then an instruction to start radiographing is input.

If there is an instruction to start radiographing through the input unit 4, radiographing of a plurality of radiological images is performed (step ST2). Specifically, first, the control unit 2a reads an angle θ0 which defines a radiographing distance set in advance and outputs the information of the read angle θ0 to the arm controller 31. In addition, in the first embodiment, θ0 = 4° is stored in advance as the information of the angle θ0 at this time. However, an operator may set an arbitrary angle of convergence through the input unit 4 without being limited to this.

Then, the arm controller 31 receives the information of the angle θ0 output from the control unit 2a. According to this information, first, the arm controller 31 outputs a control signal to make the position of the arm unit 13 become an initial position most inclined with respect to the radiography platform 14.

Then, in a state where the arm unit 13 is at the initial position according to the control signal output from the arm controller 31, the control unit 2a outputs control signals to the radiation source controller 32 and the detector controller 33 in order to perform irradiation and reading of a radiological image signal. According to this control signal, radiation is radiated from the radiation source 17, a radiological image obtained by radiographing the breast M from the initial position is detected by the radiation detector 15, a radiological image signal is read from the radiation detector 15 by the detector controller 33, and predetermined signal processing is performed on the radiological image signal. Then, the result is stored in the radiological image storage unit 2b of the computer 2.

Then, the arm controller 31 outputs a control signal to make the arm unit 13 rotate by +θ° from the initial position. That is, in the first embodiment, the arm controller 31 outputs a control signal to make the arm unit 13 rotate by 4° from the initial position in a direction toward the end position at which the final radiographing is performed. Then, in a state where the arm unit 13 has rotated by 4° according to the control signal output from the arm controller 31, the control unit 2a outputs control signals to the radiation source controller 32 and the detector controller 33 in order to perform irradiation and reading of a radiological image signal.

Then, the controller 2a outputs control signals to the radiation source controller 32 and the detector controller 33 in order to perform irradiation and reading of a radiological image signal. According to this control signal, radiation is radiated from the radiation source 17, a radiological image obtained by radiographing the breast M from the position rotated by 4° from the initial position is detected by the radiation detector 15, a radiological image signal is read by the detector controller 33, and predetermined signal processing is performed. Then, the result is stored in the radiological image storage unit 2b of the computer 2.

Then, a plurality of radiological images is stored in the radiological image storage unit 2b by repeating the above-described processing until the arm unit 13 rotates up to the end position.

Then, the feature amount calculation unit 2d calculates the feature amount representing the image quality of each of the plurality of radiological images (step ST3), and the determination unit 2e determines whether or not an abnormal radiological image with different image quality is present in the plurality of radiological images (step ST4). If YES in step ST4, the determination unit 2e outputs the determination result to the reconstruction unit 2c and the warning unit 2f (step ST5). The warning unit 2f gives a warning that there is an abnormal radiological image based on the determination result (step ST6). In addition, the reconstruction unit 2c generates a tomographic image by performing reconstruction after excluding the abnormal radiological image from the plurality of radiological images (step ST7).

On the other hand, if NO in step ST4, the determination unit 2e outputs the determination result only to the reconstruction unit 2c (step ST8). The reconstruction unit 2c generates a tomographic image by performing reconstruction using all radiological images (step ST9).

In addition, the display control unit 2g displays the generated tomographic image on the monitor 3 (step ST10), and ends the processing.

Thus, in the first embodiment, the feature amounts representing respective image qualities of a plurality of radiological images are calculated, the feature amounts of the plurality of radiological images are compared, it is determined based on the comparison result whether or not an abnormal radiological image with different image quality is present in the plurality of radiological images, and the abnormal radiological image is excluded from the plurality of radiological images to generate a tomographic image when there is an abnormal radiological image. For this reason, a radiological image with different image quality from others is not used for reconstruction. As a result, deterioration of the image quality of a tomographic image can be prevented.

In addition, the presence of an abnormal radiological image can be notified by giving a warning when there is an abnormal radiological image.

Next, a second embodiment of the present invention will be described. In addition, a radiological image radiographing apparatus according to a second embodiment has the same configuration as the radiological image radiographing apparatus according to the first embodiment, and only the processing performed is different. Accordingly, detailed explanation of the configuration will be omitted herein. In the first embodiment described above, when there is an abnormal radiological image, the reconstruction unit 2c performs reconstruction after excluding an abnormal radiological image. In the second embodiment, however, reconstruction is stopped when there is an abnormal radiological image, which is different from the first embodiment.

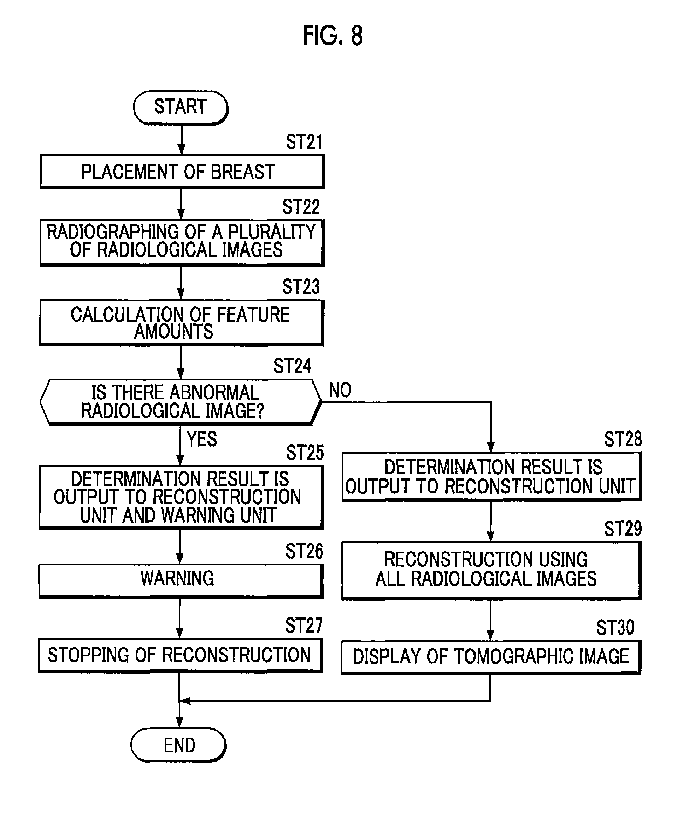

Next, processing performed in the second embodiment will be described. Fig. 8 is a flow chart showing the processing performed in the second embodiment. In addition, since processing of steps ST21 to ST24 and ST28 to ST30 in the flow chart shown in Fig. 8 is the same as the processing of steps ST1 to ST4 and ST8 to ST10 in the flow chart shown in Fig. 7, detailed explanation thereof will be omitted herein. If YES in step ST24, the determination unit 2e outputs the determination result to the reconstruction unit 2c and the warning unit 2f (step ST25). The warning unit 2f gives a warning that there is an abnormal radiological image based on the determination result (step ST26). In this case, information indicating that a tomographic image is not generated is included in the warning. In addition, the reconstruction unit 2c stops the reconstruction (step ST27), and ends the processing.

Thus, in the second embodiment, a radiological image with different image quality from others is not used for reconstruction since reconstruction is stopped when an abnormal radiological image is included in a plurality of radiological images. As a result, deterioration of the image quality of a tomographic image, which is caused by the generation of a tomographic image using an abnormal radiological image, can be prevented.

Next, a third embodiment of the present invention will be described. In addition, a radiological image radiographing apparatus according to a third embodiment has the same configuration as the radiological image radiographing apparatus according to the first embodiment except that a monitor 3A (display unit that displays a stereoscopic image)capable of performing three-dimensional display of a stereo image. Accordingly, detailed explanation thereof will be omitted herein. The radiological image radiographing apparatus according to the third embodiment is different from the radiological image radiographing apparatus according to the first embodiment in that radiographing of two radiological images for displaying a stereo image is performed instead of tomosynthesis and the display control unit 2g displays the stereo image based on the two radiological images on the monitor 3A without using the reconstruction unit 2c.

The monitor 3A is configured to be able to perform three-dimensional display of a stereo image using the two radiological images output from the computer 2. As an example of the three-dimensional display method of the monitor 3A, a method may be adopted in which two radiological images are displayed using two screens and one of the radiological images is incident on the right eye of an observer and the other radiological image is incident on the left eye of the observer using a half mirror, a polarization glass, and the like to thereby display a stereo image. In addition, a method of displaying a stereo image by superimposing two radiological images and making these radiological images observable with a polarization glass may also be used. In addition, the monitor 3A may be formed by a 3D liquid crystal display, and a method by which stereoscopic viewing of two radiological images is possible, such as a parallax barrier method and a lenticular method, may be used.

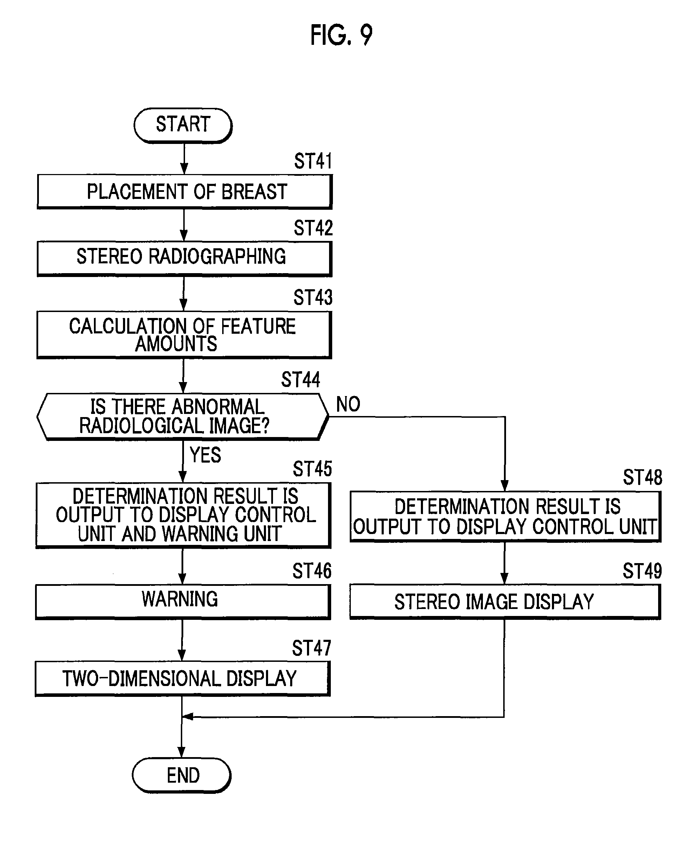

Next, processing performed in the third embodiment will be described. Fig. 9 is a flow chart showing the processing performed in the third embodiment. First, the breast M of a patient is placed on the radiography platform 14 and is compressed with predetermined pressure by the compression plate 18 (step ST41). Then, various radiographing conditions are input through the input unit 4 and then an instruction to start radiographing is input.

If there is an instruction to start radiographing through the input unit 4, radiographing (stereo radiographing) of two radiological images for displaying a stereo image is performed (step ST42). Specifically, first, the control unit 2a reads an angle of convergence θ1 for radiographing of a stereo image set in advance and outputs the information of the read angle θ1 to the arm controller 31. In addition, in the third embodiment, θ1 = ±4° is stored in advance as the information of the angle θ1 at this time. However, an operator may set an arbitrary angle of convergence through the input unit 4 without being limited to this.

Then, the arm controller 31 receives the information of the angle of convergence θ1 output from the control unit 2a. According to this information, first, the arm controller 31 outputs a control signal to rotate the position of the arm unit 13 by +θ1º with respect to the radiography platform 14. That is, in the third embodiment, the arm controller 31 outputs a control signal to rotate the arm unit 13 by +4° in a direction perpendicular to the radiography platform 14.

Then, the arm unit 13 rotates by +4° according to the control signal output from the arm controller 31. Then, the control unit 2a outputs control signals to the radiation source controller 32 and the detector controller 33 in order to perform irradiation and reading of a radiological image signal. According to this control signal, radiation is radiated from the radiation source 17, a radiological image obtained by radiographing the breast M from the initial position is detected by the radiation detector 15, a radiological image signal is read from the radiation detector 15 by the detector controller 33, and predetermined signal processing is performed on the radiological image signal. Then, the result is stored in the radiological image storage unit 2b of the computer 2. In addition, the radiological image signal stored in the radiological image storage unit 2b by this radiographing expresses a radiological image for a right eye GR.

Then, the arm controller 31 returns the arm unit 13 to its initial position and then outputs a control signal to rotate the arm unit 13 by -θ1° in a direction perpendicular to the radiography platform 14. That is, in the third embodiment, the arm controller 31 outputs a control signal to rotate the arm unit 13 by +4° in a direction perpendicular to the radiography platform 14.

Then, the arm unit 13 rotates by -4° according to the control signal output from the arm controller 31. Then, the control unit 2a outputs control signals to the radiation source controller 32 and the detector controller 33 in order to perform irradiation and reading of a radiological image. According to this control signal, radiation is radiated from the radiation source 17, a radiological image obtained by radiographing the breast M from the direction of -4° is detected by the radiation detector 15, a radiological image signal is read by the detector controller 33, and predetermined signal processing is performed. Then, the result is stored in the radiological image storage unit 2b of the computer 2. In addition, the radiological image signal stored in the radiological image storage unit 2b by this radiographing expresses a radiological image for a left eye GL.

Then, the feature amount calculation unit 2d calculates the feature amount of each of the two radiological images GR and GL (step ST43), and the determination unit 2e determines whether or not an abnormal radiological image with different image quality is present in the two radiological images GR and GL (step ST44). In addition, the determination of the determination unit 2e in the third embodiment compares the feature amounts representing respective image qualities of the two radiological images GR and GL, and to determine that there is an abnormal radiological image when the feature amounts are different by an amount exceeding a predetermined threshold value.

If YES in step ST44, the determination unit 2e outputs the determination result to the warning unit 2f and the display control unit 2g (step ST45). The warning unit 2f gives a warning that there is an abnormal radiological image based on the determination result (step ST46). In this case, information indicating that a stereo image is not displayed is included in the warning. In addition, the display control unit 2g displays either or both of the two radiological images GR and GL on the monitor 3A in a two-dimensional manner instead of a stereo image (step ST47), and ends the processing.

On the other hand, if NO in step ST44, the determination unit 2e outputs the determination result to the display control unit 2g (step ST48). The display control unit 2g displays a stereo image based on the two radiological images GR and GL on the monitor 3A (step ST49), and ends the processing.

Thus, in the third embodiment, a radiological image with different image quality from others is not used for display of a stereo image, since the display of a stereo image is stopped when an abnormal radiological image is included in a plurality of radiological images. As a result, deterioration of the image quality of a stereo image, which is caused by the display of a stereo image using an abnormal radiological image, can be prevented.

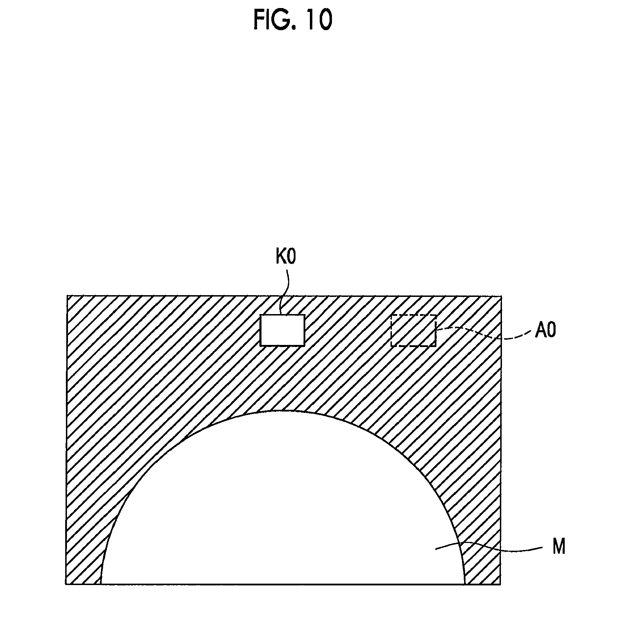

In addition, although the density histogram, the spatial frequency, and the edge information are used as the feature amount representing the image quality of a radiological image in the first to third embodiments described above, information regarding the S/N ratio of a radiological image or the contrast of a radiological image may also be used as the feature amount. Hereinafter, calculation of the information regarding the S/N ratio or the contrast will be described. Fig. 10 is a view for explaining the calculation of the information regarding the S/N ratio or the contrast. In addition, when the information regarding the S/N ratio or the contrast is used as the feature amount, a marker, such as metal through which radiation is not transmitted, is radiographed together with the breast M. In addition, a marker used for alignment when reconstructing a plurality of radiological images, may be used as the marker described above. In this case, a marker image K0 is included in each radiological image together with an image of the breast M, as shown in Fig. 10. In addition, the marker image K0 has a low density (that is, high brightness).

Here, for the S/N ratio, the average value and standard deviation of pixel values in the marker image K0 may be calculated, and a ratio between the average value and standard deviation of pixel values may be calculated.

On the other hand, when a reference region A0 with a predetermined size is set in an element dropping out portion (shaded portion in Fig. 10) corresponding to a portion in a radiological image, to which radiation is directly radiated, the reference region A0 has a high density (that is, low brightness). Accordingly, the contrast information can be calculated as a difference between the average value of the pixel values in the marker image K0 and the average value of the pixel values in the reference region A0.

Here, if the amount of radiation radiated when acquiring a certain radiological image becomes low, the S/N ratio of the radiological image becomes high and the contrast becomes low. For this reason, it can also be determined whether or not an abnormal radiological image is present in a plurality of radiological images by using the S/N ratio and the contrast information. That is, a radiological image with a higher S/N ratio than other radiological images can be identified as an abnormal radiological image, and a radiological image with the lower contrast information than other radiological images can be identified as an abnormal radiological image.

Moreover, in the first to third embodiments, any of the density histogram, the spatial frequency, and the edge information is calculated as a feature amount representing the image quality of a radiological image. However, it is also possible to calculate a plurality of feature amounts representing the image quality of a radiological image including not only the density histogram, the spatial frequency, and the edge information but also the information regarding the S/N ratio of a radiological image and the contrast of a radiological image described above ,and to determine whether or not an abnormal radiological image with different image quality is present in the plurality of radiological images using one of the plurality of calculated feature amounts.

In addition, in the first and second embodiments described above, a tomographic image is generated by reconstructing a plurality of radiological images acquired by tomosynthesis. However, the tomographic image may also be generated by disposing a radiation source and a radiation detector opposite each other with a subject located therebetween, rotating the radiation source and the radiation detector around the subject, radiating radiation from various angles to capture a plurality of radiological images, and performing CT radiographing to display an arbitrary cross section by reconstructing a tomographic image using the radiological image from each of the angles.

In addition, although the radiological image radiographing apparatuses according to the first to third embodiments of the present invention are apparatuses which radiograph a radiological image of a breast, a subject is not limited to the breast. For example, a radiological image radiographing apparatus which radiographs a chest, a head, and the like may also be used.

## Claims

1. A radiological image radiographing apparatus comprising:
an irradiation unit that radiates radiation to a subject;
a radiation detection unit that detects radiation, which is radiated from the irradiation unit to be then transmitted through the subject, to acquire a radiological image of the subject;
a radiographing control unit that controls the irradiation unit and the radiation detection unit to radiate the radiation to the subject from a plurality of radiographing directions and to acquire a plurality of radiological images with different directions of radiographing by irradiation of the radiation;
a feature amount calculation unit that calculates feature amounts representing an image quality of each of the plurality of radiological images; and
a determination unit that compares the feature amounts of the plurality of radiological images and based on the comparison result, and then determines whether or not an abnormal radiological image with different image quality is present in the plurality of radiological images.

2. The radiological image radiographing apparatus according to claim 1,
wherein the feature amount calculation unit calculates the feature amounts based on at least one of density histograms, spatial frequencies, edge information, S/N ratios, and contrast of the plurality of radiological images.

3. The radiological image radiographing apparatus according to claim 1 or 2,
wherein the determination unit determines whether or not the abnormal radiological image is present in the plurality of radiological images by identifying a radiological image with feature amounts, which are different from feature amounts of other radiological images by a predetermined threshold value or more, as the abnormal radiological image.

4. The radiological image radiographing apparatus according to any one of claims 1 to 3, further comprising:
a reconstruction unit that generates a tomographic image on a desired sectional surface of the subject by reconstructing the plurality of radiological images when the plurality of radiological images are acquired in order to generate a tomographic image.

5. The radiological image radiographing apparatus according to claim 4,
wherein, when the abnormal radiological image is present, the reconstruction unit generates the tomographic image by reconstructing other radiological images excluding the abnormal radiological image in the plurality of radiological images.

6. The radiological image radiographing apparatus according to claim 4,
wherein the reconstruction unit stops the reconstruction when the abnormal radiological image is present.

7. The radiological image radiographing apparatus according to any one of claims 1 to 3, further comprising:
a display unit that, when the plurality of radiological images are two radiological images acquired in order to display a stereoscopic image, displays the stereoscopic image based on the two radiological images.

8. The radiological image radiographing apparatus according to claim 7,
wherein the display unit stops display of the stereoscopic image when the abnormal radiological image is present.

9. The radiological image radiographing apparatus according to any one of claims 1 to 8, further comprising:
a warning unit that gives a warning when the abnormal radiological image is present.

10. A radiological image radiographing method in a radiological image radiographing apparatus including an irradiation unit that radiates radiation to a subject, a radiation detection unit that detects radiation, which is radiated from the irradiation unit to be transmitted through the subject, to acquire a radiological image of the subject, and a radiographing control unit that controls the irradiation unit and the radiation detection unit to radiate the radiation to the subject from a plurality of radiographing directions and to acquire a plurality of radiological images with different directions of radiographing by irradiation of the radiation, the radiological image radiographing method comprising:
calculating feature amounts indicating respective image qualities of the plurality of radiological images;
comparing the feature amounts of the plurality of radiological images; and
determining, based on the comparison result, whether or not an abnormal radiological image with different image quality is present in the plurality of radiological images.
